# EUROPEAN PATENT SPECIFICATION

(11) **EP 3 335 185 B1**
(45) Date of publication and mention of the grant of the patent: **09.04.2025**
(21) Application number: 16837585.5
(22) Date of filing: 12.08.2016
(51) Int. Cl.: G16H 15/00, G16H 20/17, G16H 40/63, A61B 5/00, A61B 5/145

(54) **DIABETES MANAGEMENT SYSTEM WITH ALERT STATUS INTERFACE AND PATIENT PRIORITIZATION**
DIABETESMANAGEMENTSYSTEM MIT WARNSTATUSSCHNITTSTELLE UND PATIENTENPRIORISIERUNG
SYSTÈME DE GESTION DU DIABÈTE AYANT UNE INTERFACE D'ÉTAT D'ALERTE ET UNE PRIORISATION DES PATIENTS

(30) Priority: 14.08.2015 US 201562205351 P
(43) Date of publication of application: 20.06.2018
(73) Proprietor: Roche Diagnostics GmbH, 68305 Mannheim (DE); F. Hoffmann-La Roche AG, 4070 Basel (CH)
(72) Inventor: BUSKIRK, Ann, Carmel, IN 46032 (US); DAJANI, Samer, Carmel, IN 46033 (US); GALLEY, Paul, Indianapolis, IN 46229 (US); GEJDOS, Igor, Indianapolis, IN 46240 (US); KOEHLER, Matthias, 69514 Laudenbach (DE); LONG, Michael L., Pendleton, IN 46064 (US); REES, Christen, Indianapolis, IN 46256 (US); CARLSGAARD, Eric S., Zionsville, IN 46077 (US)
(74) Representative: Bittner, Thomas L.
(86) International application number: PCT/US2016/046783
(87) International publication number: WO 2017/030961

(56) References cited:
- US-A1- 2008 071 580
- US-A1- 2012 172 694
- US-A1- 2013 035 871
- US-A1- 2013 035 871
- US-A1- 2014 066 844
- US-A1- 2014 344 280
- US-A1- 2014 344 280
- US-A1- 2015 095 042

## Description

The present disclosure relates a computer implemented method for prioritizing patients associated with a healthcare provider.

### BACKGROUND

Management of chronic illness, like diabetes, involves collecting and analyzing large amounts of data. Such data may be acquired from medical devices, personal healthcare devices, patient recorded information, and test results. For people with diabetes, successful management requires monitoring one's blood glucose level. As a tool for understanding changes in one's blood glucose level, a patient may access and run a variety of reports which aggregate and present diagnostic data over preset time periods, such as days, weeks, or even months. However, interfaces are needed to alert and inform a healthcare provider or a person suffering diabetes of possible harmful changes in the patient's blood glucose level.

This section provides background information related to the present disclosure which is not necessarily prior art.

US 2013/0035871 A1 discloses systems and methods for detecting and reporting patterns in blood glucose measurements. The pattern data can be based on detecting frequency and severity of measurement data in clinically risky ranges, wherein data can be evaluated by using weight factors.

US 2014/0344280 A1 discloses methods to evaluate continuous glucose monitoring (CGM) data and insulin delivery data to generate projected alarms related to a projected glucose levels. Profiles of CGM data are created for use in tuning patient-specific insulin data, such at basal rate, carb ratio, and insulin sensitivity. Patterns in the data profiles are determined to recommend changes to patient-specific insulin data.

US 2015/0095042 A1 discloses a blood glucose (bG) measurement engine to selectively measure bG levels in blood samples input to a handheld diabetes management device during a certain period of days during which the patient input at least two blood samples. A value is calculated based on the bG levels of blood samples input during the period, and based on the value, the patient is classified as having a first, second, or third risk of being hypoglycemic in the future.

US 2012/172694 A1 discloses methods and apparatuses for determination and application of a unidimensional metric for assessing a patient's glycemic health. In one particular implementation, a computed metric may be used to balance short-term and long-term risks associated with a particular therapy. In another implementation, a computed unidimensional metric may be used to balance risks of hyperglycemia and hypoglycemia.

### SUMMARY

A computer implemented method for prioritizing patients associated with a healthcare provider according to the independent claim 1 is provided. Embodiments are set out in dependent claims.

### DRAWINGS

The drawings described herein are for illustrative purposes only of selected embodiments and not all possible implementations.
FIG. 1 illustrates a diabetes management system in communication with multiple devices;
FIG. 2 is a functional block diagram of the diabetes management system;
FIG. 3 illustrates an example of a patient summary interface provided by the diabetes management system;
FIG. 4 is a flowchart illustrating an example routine for determining a glucose alert for a patient;
FIGS. 5A, 5B, 5C, 5D, and 5E are tables illustrating example guidelines for determining various glucose conditions;
FIG. 6 illustrates an example of an alert setting interface provided by the diabetes management system;
FIG. 7 illustrates an example of a healthcare provider welcome interface provided by the diabetes management system;
FIGS. 8A, 8B, and 8C are examples of a condition weight table, a state weight table, and a condition-state weight table;
FIG. 9 is an example of a group criteria table for sorting patients based on a total alert weight; and
FIG. 10 is a flowchart illustrating an example routine for sorting and prioritizing patients.

Corresponding reference numerals indicate corresponding parts throughout the several views of the drawings.

### DETAILED DESCRIPTION

The present disclosure will now be described more fully with reference to the accompanying drawings.

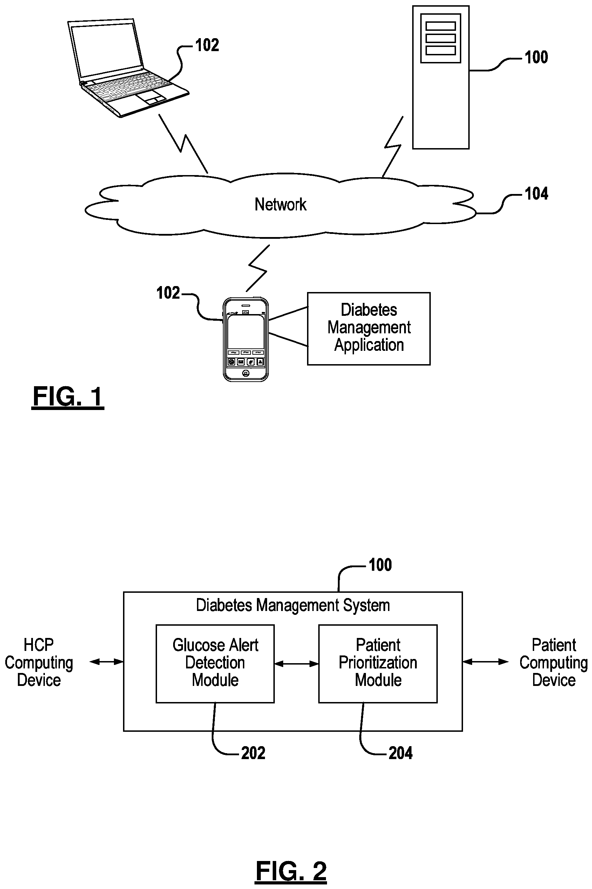

With reference to FIGS. 1 and 2, a diabetes management system (DMS) 100 may be housed in one or more servers and includes software tools that enable users to manage diabetes. A user, such as a person suffering from diabetes (i.e., patient) and/or the healthcare provider for the patient, may access the software tools supported by the diabetes management system 100 by way of a computing device 102. For example, the diabetes management system 100 and the computing device 102 may exchange information via a communication link to a communication network 104, such as the Internet. The computing device 102 may include: a laptop; a portable computing device, such as a smartphone and/or tablet having a diabetes management application residing in and executed by the portable computing device; a medical device, such as blood glucose meter; and/or other suitable device that exchanges and processes information.

Using the computing device 102, the healthcare provider (i.e., HCP) may create a user account and register with the diabetes management system 100 as a healthcare provider member. For example, through a user interface supported by the diabetes management system 100, the healthcare provider may create a profile that is stored by the diabetes management system 100. The profile may include information for identifying the healthcare provider and information regarding patients associated with the healthcare provider.

Similarly, the patient may establish a user account and register as a client member with the diabetes management system 100. As an example, through a user interface supported by the diabetes management system 100, the patient creates a profile that is stored by the diabetes management system 100. Once registered, the patient may have access to tools supported by the diabetes management system 100, such as a bolus calculator and a logbook for storing blood glucose (bG) measurements. The bG measurement may include a numerical measurement, a unit of measurement (e.g., mg/dl), a timestamp, and a comment (e.g. before breakfast or after exercise). The bG measurement may be entered manually by the patient by way of a user interface on computing device 102 or may be transmitted by a glucose measurement device to the DMS 100.

The patient may also authorize one or more healthcare providers registered with the diabetes management system 100 to access information related to the patient. For example the patient may send an invitation to a selected healthcare provider member to link with the patient. Once the selected healthcare provider member accepts the patient's invitation, the healthcare provider may view information related to the patient, such as bG measurements. Alternately, the healthcare provider may send an invitation to the patient to link with the healthcare provider. Once the patient accepts the healthcare provider's invitation, the healthcare provider may view information related to the patient, such as bG measurements.

The diabetes management system 100 enables the patient and the healthcare provider to monitor the patient's bG measurements. For instance, the diabetes management system 100 includes a glucose alert detection module 202 and a patient prioritization module 204. The glucose alert detection module 202 may be used by the patient and/or the healthcare provider for monitoring a glucose condition of the patient and for notifying the patient and/or healthcare provider of an alert status regarding the patient's blood glucose level. The patient prioritization module 204 may be used by the healthcare provider to sort and prioritize multiple patients associated with the healthcare provider based on the alert status for each of the patients.

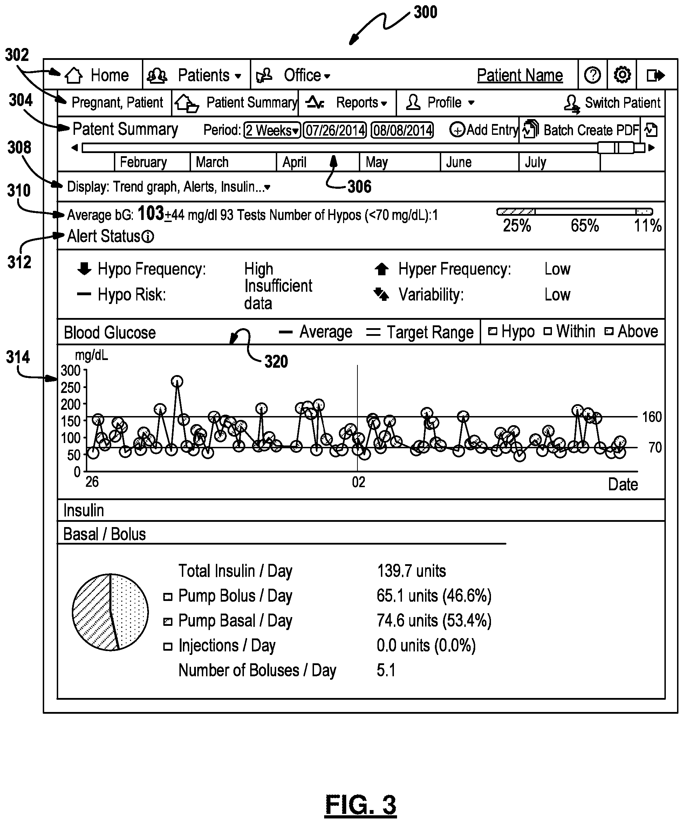

The glucose alert detection module 202 analyzes the patient's bG measurements to determine a glucose condition for a specified enquiry period and to generate an alert status for a patient summary interface. As an example, FIG. 3 illustrates a patient summary interface 300 that is generated by the diabetes management system 100. The patient summary interface 300 includes a menu section 302, a title bar 304, an enquiry period selection section 306, a display selection bar 308, a glucose synopsis bar 310, an alert status section 312, and a digest section 314.

The menu section 302 displays drop down menus and tabs accessible by the user and may be continuously displayed by the diabetes management system 100. The title bar 304 display contextual information regarding the interface currently being displayed. For example, in FIG. 3, the title bar 304 identifies the interface 300 as the "Patient Summary."

The enquiry period selection section 306 displays data input interfaces that enable the user to input or select the enquiry period. The enquiry period is a time period for which the glucose condition is analyzed. The enquiry period selection section 306 may include a drop down menu for displaying preset time periods (e.g., "2 Weeks" in FIG. 3) and text input interfaces for inputting specified dates that define the enquiry period (e.g., "07/26/2014" and "08/08/2014" in FIG. 3). The enquiry period may be configured to span a minimum or maximum time period. For example, the user may be able to set a time period between 1 to 10 weeks or 3-days to 12 weeks or other suitable time period. The enquiry period selection section may be referred to as a first section of the patient summary interface 300.

The display selection bar 308 includes a drop down menu that lists various graphs that may be displayed in the digest section. For example, the user may select the standard day graph from the drop down menu in order to view the graph in the digest section 314 or unselects the graph to remove it from the digest section 314.

The glucose synopsis bar 310 displays textual information that provides a summary of the patent's bG level during the enquiry period. For example, the glucose synopsis bar 310 may include information indicative of an average bG, average number of bG tests, number of hypoglycemic occurrences. While FIG. 3 illustrates specific information displayed in the glucose synopsis bar 310, other suitable glucose information may be displayed, such as number of hyperglycemic occurrences, and is not limited to the information illustrated.

The alert status section 312 displays textual information regarding one or more glucose conditions analyzed with respect to the enquiry period and an alert status for the patient based on the glucose conditions. As described in detail below, the one or more glucose conditions is indicative of a bG level of the patient and may include: hypoglycemia frequency, hypoglycemia risk, hyperglycemia frequency, hyperglycemia risk, and/or blood glucose variability.

The digest section 314 of the patient summary interface 300 displays information indicative of the patient's bG measurements during the enquiry period. For example, FIG. 3 illustrates a graph 320 that conveys the patient's bG measurements for the enquiry period. The digest section 314 may also include information regarding different activities that may affect the patient's bG measurement such as insulin dosage, meal time, calorie consumption, etc. The digest section 314 may also be referred to as a second section of the patient summary interface 300.

The glucose alert detection module 202 determines the glucose conditions based on bG measurements taken during the enquiry period and determines a state of the glucose condition based on an alert threshold. The state of the glucose condition is determined as: HIGH, LOW, or not available due to insufficient data. A HIGH state indicates that the glucose condition is excessive or, in other words, over or outside of an acceptable range. A LOW state indicates that the glucose condition is normal or, in other words, within the acceptable range. The state of the glucose condition may include other states and is not limited to "HIGH", "LOW', and "Insufficient Data".

In some embodiments, the alert status section 312 may only be displayed by the diabetes management system 100 when the glucose alert detection module 202 determines that at least one of the glucose conditions is outside an acceptable range (i.e., HIGH). For example, FIG. 3 illustrates a situation in which the state of the bG hypoglycemia frequency (i.e., "Hypo Frequency") is HIGH for the enquiry period. The alert status section 312 is located between the enquiry period selection section 306 and the digest section 314 of the patient summary interface 300. The alert status section 312 may also display textual information regarding the state of the other glucose conditions (e.g., "Hypo risk", "Hyper Frequency", and "Variability").

The glucose alert detection module 202 may be configured to determine the glucose conditions periodically, when a new bG measurement is received, or when the user activates a particular user interface, such as the patient summary interface 300. For each of the glucose conditions, the glucose alert detection module 202 stores at least one glucose threshold and an alert threshold. The glucose threshold assess whether a given bG level is within a desirable range. The alert threshold determines the state of the glucose condition or, in other words, whether the glucose condition is within an acceptable range.

By way of example, when the glucose condition is a bG hypoglycemia frequency, the glucose alert detection module 202 determines the total number of occurrences in which the patient's bG measurement is below a bG hypo-target threshold. More particularly, the glucose alert detection module 202 compares a given bG measurement to the bG hypo-target threshold (e.g., 70 mg/dL or other suitable threshold). The bG hypo-target threshold may be set by the user or by the diabetes management system 100. If the given bG measurement is less than or equal to the bG hypo-target threshold, the glucose alert detection module 202 determines the occurrence of a hypoglycemic condition. If the given bG measurement is greater than the bG hypo-target threshold, the glucose alert detection module 202 determines the patient is not hypoglycemic. The glucose alert detection module 202 performs the comparison for each bG measurement taken in the enquiry period, which are stored in the diabetes management system 100.

The glucose alert detection module 202 then determines the total number of hypoglycemic occurrences for the enquiry period. The state of the hypoglycemia frequency may be determined based on the total number of hypo-occurrences and/or by a hypo-occurrence percentage. For the hypo-occurrence percentage, the glucose alert detection module 202 divides the total number of hypoglycemic occurrences by the total number of bG measurements taken during the enquiry period, and multiplies the ratio by 100.

To determine the state of the hypoglycemia frequency for the patient, the glucose alert detection module 202 compares the total hypo-occurrences and/or the hypo-occurrence percentage to respective alert thresholds. For example, the hypo-occurrence percentage is compared to an occurrence percentage alert threshold and the total hypo-occurrences is compared to an occurrence number threshold. If the hypo-occurrence percentage is greater than or equal to the occurrence percentage threshold, the hypoglycemia frequency is considered over an acceptable range and the state of the hypoglycemia frequency is determined as HIGH. If the hypo-occurrence percentage is less than the occurrence percentage threshold, the hypoglycemia frequency is considered within the acceptable range and the state of the hypoglycemia frequency is determined as LOW. Similarly, if the total hypo-occurrences is greater than or equal to the occurrence number threshold, the state of the hypoglycemia frequency is determined as HIGH, and if the total hypo-occurrences is less than the occurrence number threshold, the state of the hypoglycemia frequency is determined as LOW. The glucose alert detection module 202 issues an alert for the hypoglycemia frequency when at least one of the hypo-occurrence percentage or the total hypo-occurrences are determined as HIGH. It is envisioned that alerts may be further delineate by visual indicators, for example a red arrow may be placed adjacent to the glucose condition determined to be HIGH (i.e., hypofrequency in Fig. 3); whereas, an indicia adjacent to a glucose condition determined to be LOW or INSUFF DATA may be greyed out. Other types of delineations are contemplated by this disclosure.

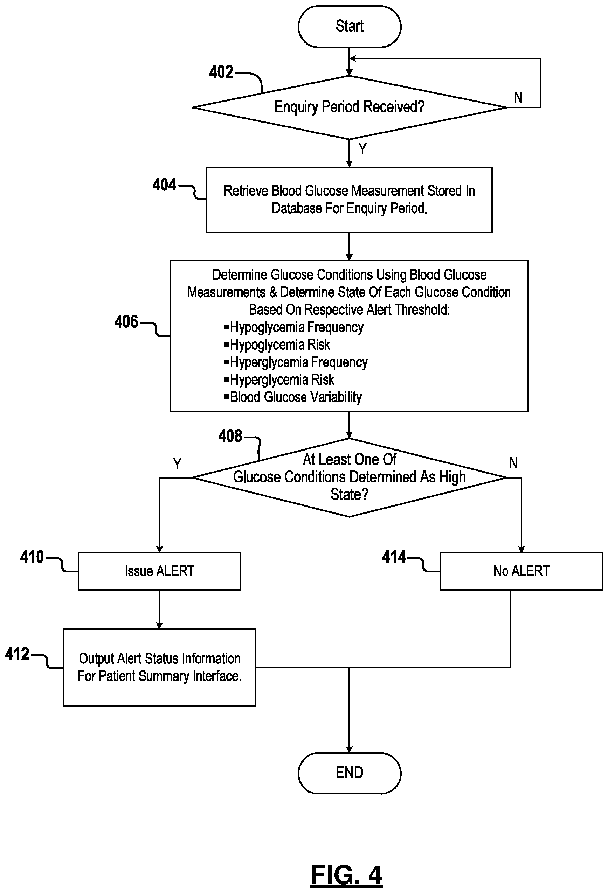

FIG. 4 illustrates a flowchart of an example glucose alert determination routine performed by the diabetes management system 100. At 402, the routine determines whether an enquiry period is received. The enquiry period may be set by the user via the patient summary interface 300 or the initial enquiry period may be set by the diabetes management system 100 (e.g., two weeks). At 404, the glucose measurements taken during the enquiry period is acquired from the database of the diabetes management system 100.

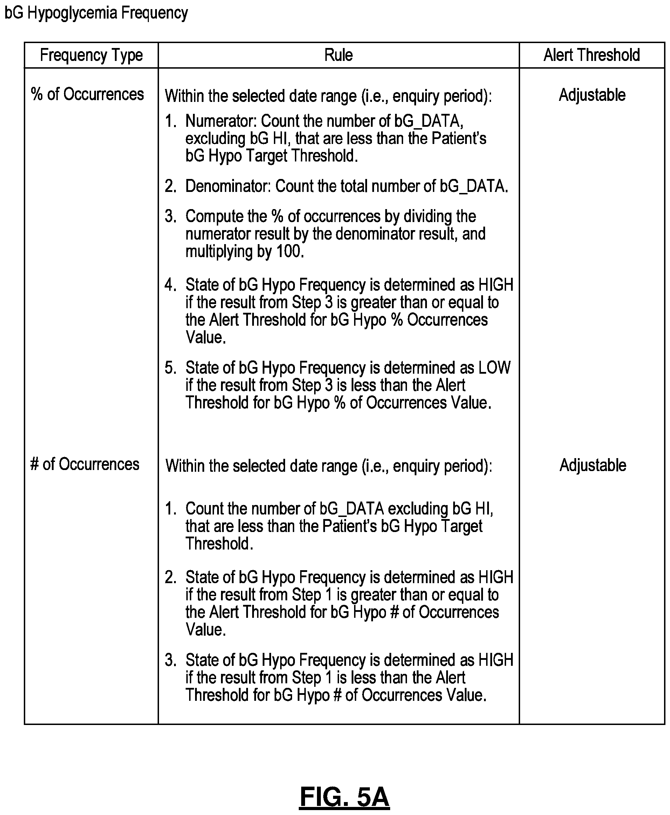

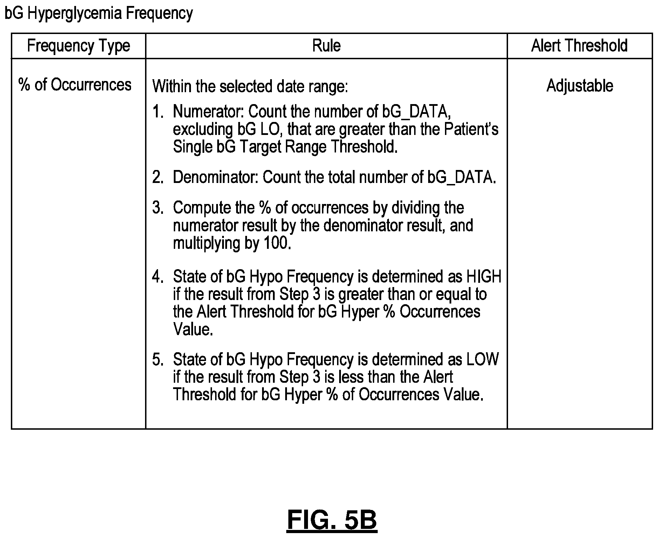

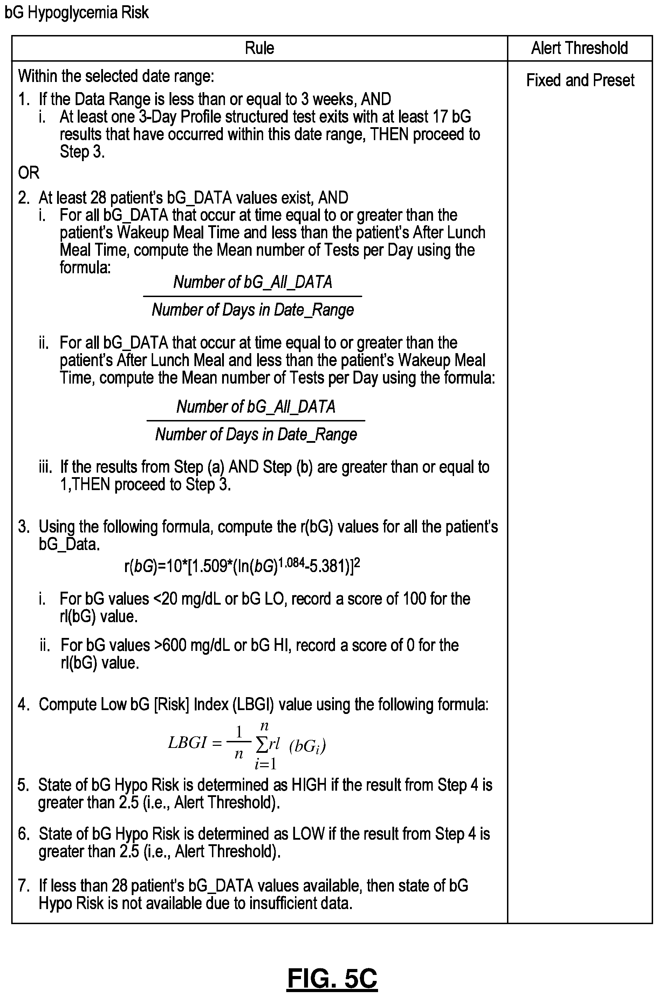

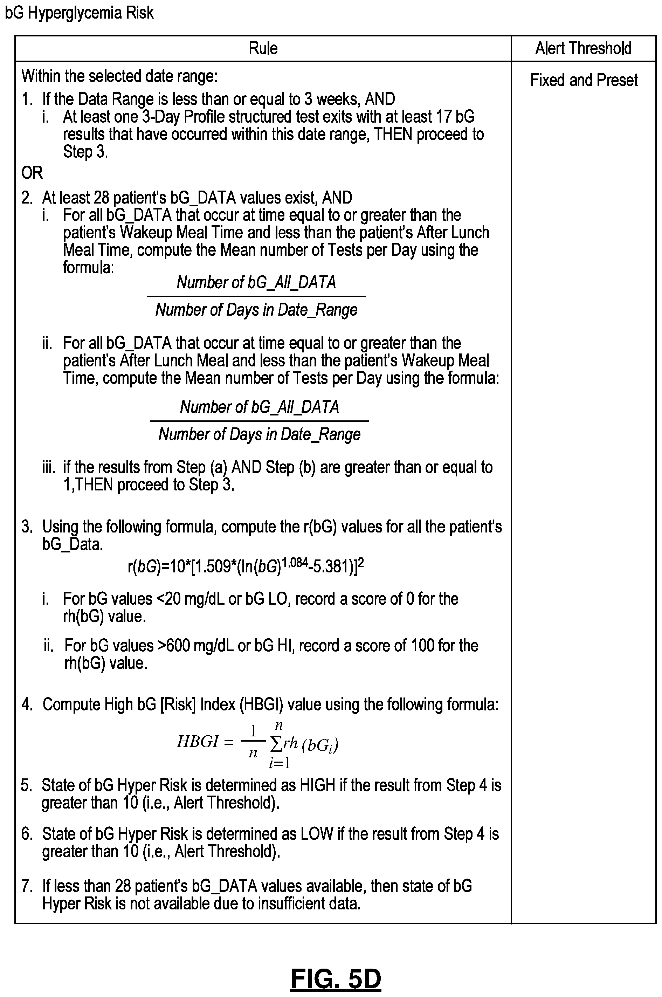

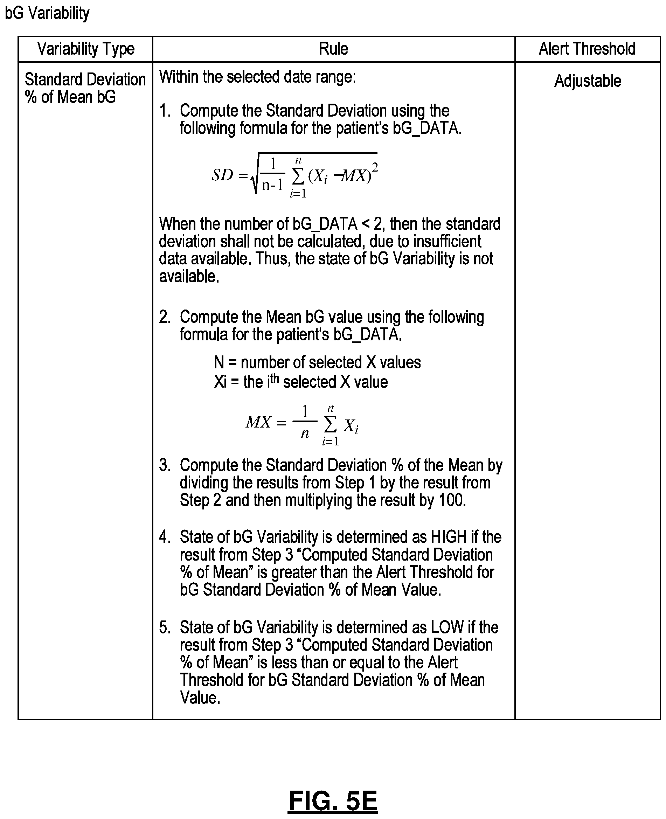

At 406, the routine determines the glucose conditions using the bG measurements and determines the state of each of the glucose conditions based on an alert threshold for respective glucose condition. For example, FIGS. 5A-5E illustrate exemplary set of guidelines that may be executed by the diabetes management system 100 for determining the glucose condition and determining the state of the glucose condition. While specific guidelines are illustrated for determining the hypoglycemia frequency, the hypoglycemia risk, the hyperglycemia frequency, the hyperglycemia risk, and/or the blood glucose variability, other guidelines and/or standards may be used to determine these glucose conditions.

At 408, the routine determines whether the state for any one of the glucose conditions is determined as HIGH or, in other words, over an acceptable range. If one of the glucose conditions is HIGH, the routine, at 410, determines that an alert should be issued and outputs, at 412, alert status information for the patient summary interface 300. The alert status information may include information identifying each of the glucose conditions and the status for each of the glucose conditions. When the alert is to be issued, the diabetes management system 100 displays the alert status section 312 in the patient summary interface 300.

If none of the glucose conditions have a HIGH state, the routine, at 414, determines no alert needs to be issued and the routine ends. Since there is no alert, the diabetes management system 100 does not display the alert status section 312 in the patient summary interface 300. In other embodiments, if no alerts need to be issued, the diabetes management system 100 may still opt to display the alert status section 312 in the patient summary interface 300. In this case, indicia adjacent each glucose condition is greyed out.

The glucose condition includes: the hypoglycemia frequency, hypoglycemia risk (LGBI), hyperglycemia frequency, hyperglycemia risk (HBGI), and blood glucose variability. However, other parameters that indicate the level or trend of the bG level may be used and is not limited to the glucose conditions described herein.

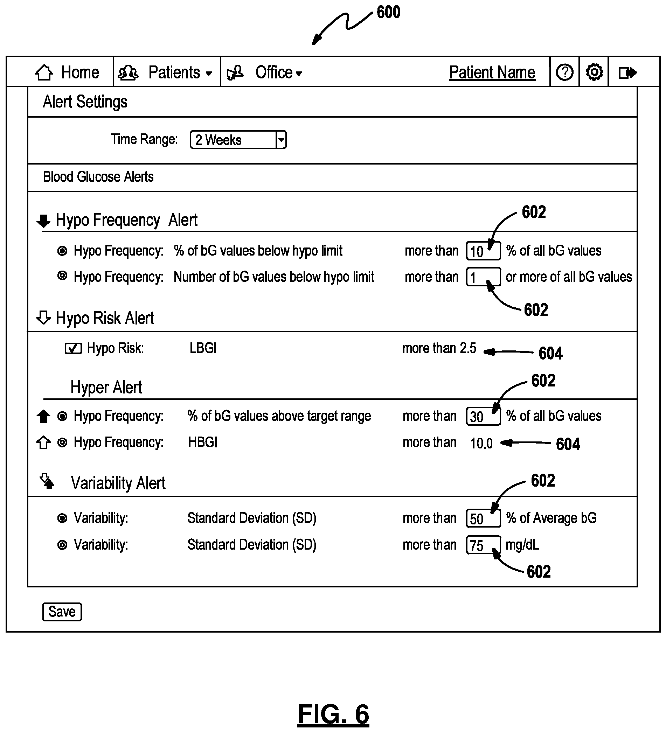

The alert threshold, which is used for determining the state of the glucose condition for the patient, may be predetermined and fixed or may be adjustable by the user. For example, FIGS. 5A-5E indicate whether the alert threshold for a respective glucose condition is fixed or adjustable. With reference to FIG. 6, the user may change an adjustable alert threshold by way of an alert setting interface 600. The alert setting interface 600 defines the different glucose conditions and the respective alert threshold. As illustrated, an alert threshold that may be adjusted is provided with an input text interface 602 (e.g., alert thresholds for hypo-frequency, hyper-frequency, and variability), and an alert threshold that is fixed includes textual information 604 to indicate the threshold (e.g., alert threshold for hypo-risk and hyper risk).

The patient and the healthcare provider may both change the alert thresholds for their respective profiles. That is, in the example embodiment, the healthcare provider may not change the alert threshold for the patient, but may be able to recommend a new threshold by notifying the patient by way of, for example, an electronic message or phone call. In another example embodiment, the alert threshold set by healthcare provider may supersede an alert threshold set by the patient.

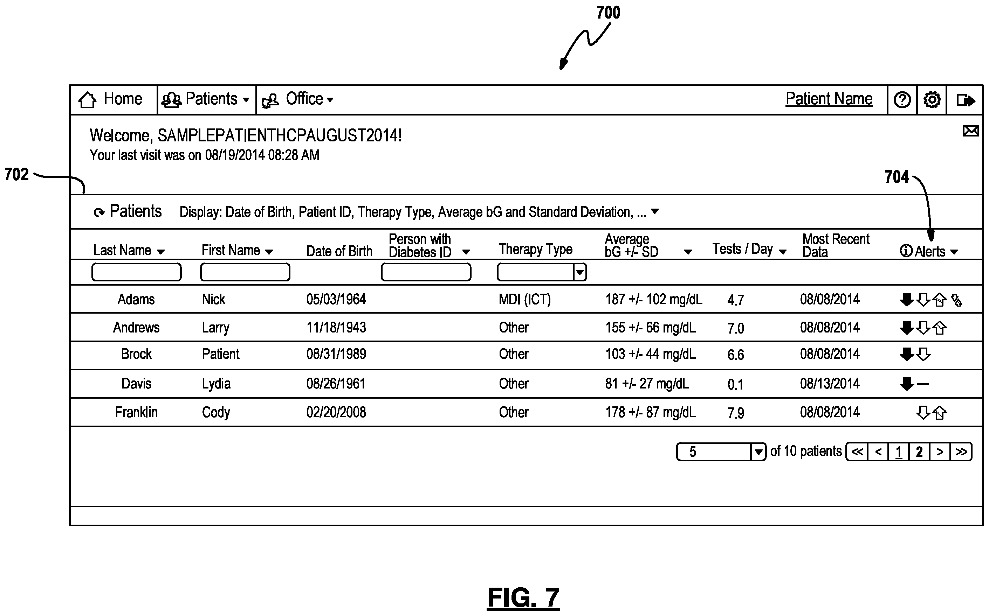

The healthcare provider may view the patient summary interface 300 or another interface that conveys similar information for a given patient. The healthcare provider may be linked with multiple patients via the diabetes management system 100. Accordingly, each patient may have different alert statuses due to one or more HIGH state glucose conditions. The patient prioritization module 204 sorts and prioritizes the healthcare provider's patients based on the alert status of each of the patient. As an example, FIG. 7 illustrates a healthcare provider (HCP) welcome interface 700 that includes a prioritized list 702 of patients determined by the patient prioritization module 204.

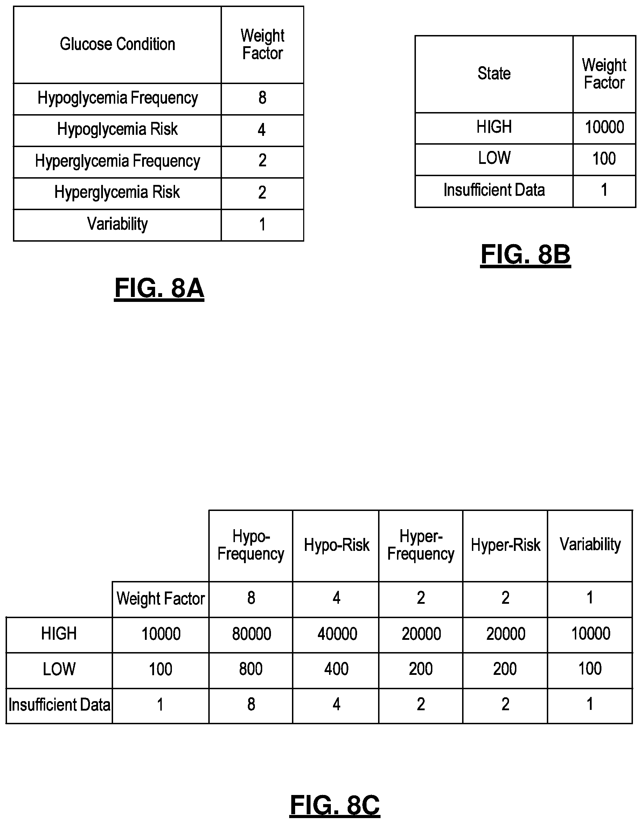

The patient prioritization module 204 utilizes an alert sort algorithm for prioritizing the patients based on the alerts status. Specifically, the alert sort algorithm pre-assigns a weight factor for each glucose condition and for each possible state of a given glucose condition. As an example, FIG. 8A illustrates a condition weight table that lists the assigned weight for each glucose condition and FIG. 8B illustrates a state weight table that lists the assigned weight for each state. In the example embodiment, the glucose conditions are weighted such that the hypoglycemia frequency is assigned the highest weight followed by the hypoglycemia risk, then the hyperglycemia frequency/risk, and then the variability. With regard to the states, the HIGH state is given the highest weight followed by the LOW state and then the insufficient data.

FIG. 8C illustrates a condition-state weight table that provides an effective weight for a particular glucose condition and state combination. The effective weight is equal to the weight factor for the glucose condition multiplied by the weight factor for the state. For example, a hypoglycemia frequency having a HIGH state has an effective weight equal to 80,000 (i.e., 8 X 10,000=80,000).

To prioritize the patients, the patient prioritization module 204 determines a total alert weight. Specifically, using the state determined for each glucose condition by the glucose prioritization module 202, the patient prioritization module 204 determines the effective weight for each glucose condition and sums the effective weight to determine the total alert weight for the patient. As an example, if the glucose prioritization module 202 determines that the hyperglycemia frequency is HIGH, the hyperglycemia risk is LOW, the hypoglycemia frequency and risk are LOW, and the variability is LOW, the patient prioritization module 204 determines the effective weights as: Hyper-Freq = 20000, Hyper-risk = 200, Hypo-Freq = 800, Hypo-Risk = 400, and Variability = 100. The total alert weight for the patient is determined as the sum of the effective weights, which is equal 21,500. The patient prioritization module 204 determines the total alert weight for each patient associated with the healthcare provider.

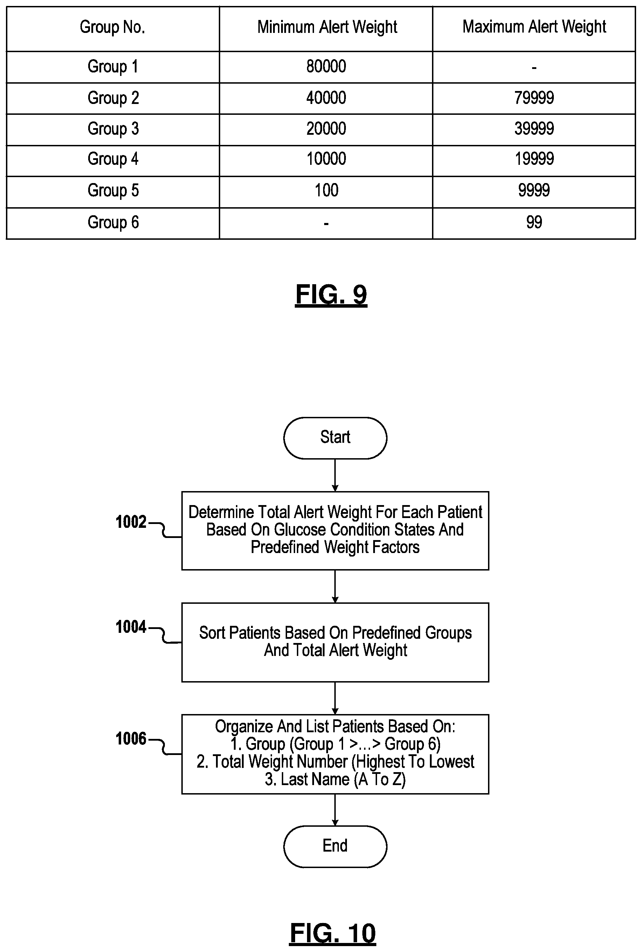

Based on the total alert weight for each of the patients, the patient prioritization module sorts the patients into different groups based on the total alert weight. For example, FIG. 9 illustrates a group criteria table that identifies multiple groups (Groups 1-6) and ranks each of the groups such that Group 1 > Group 2 > ... > Group 6. The group criteria table also provides the minimum and maximum alert weights for each group. Accordingly, based on the total alert weight for a given patient, the patient prioritization module 204 sorts the patients into one of the six groups. For example, the patient having a total alert weight of 21,500 is sorted into Group 3.

The patient prioritization module 204 then displays the patients such that the patients sorted in Group 1 are listed first, followed by the patients in Group 2, then Group 3, then Group 4, then Group 5, and finally Group 6. If there are multiple patients in each group, the patient prioritization module 204 may list the patients from highest to lowest total alert weight, and if two patients have the same total alert weight the patient prioritization module 204 may list the patients in alphabetical order.

Once sorted and prioritized, the diabetes management system 100 displays the list on the HCP welcome interface 700 as the prioritized list 702. The prioritized list 702 may also display an alert summary section 704 for each of the patient's. The alert summary 704 is indicative of the information provided in the alert status section 312 of the patient summary interface 300 if available.

FIG. 10 illustrates a flowchart of a prioritization routine performed by the diabetes management system 100. At 1002, the routine determines the total alert weight for each patient based on the state determined for each glucose condition and predefined weight factors. For example, the effective weight for each glucose condition is determined based on the predefined weight factors, and the effective weights for all of the glucose conditions is summed together to generate the total alert weight. At 1004, the routine sorts the patients based on the total alert weight. In the example embodiment, the patients are sorted into one of six groups. However, different number of groups may be defined and should not be limited to six predefined groups. Finally, at 1006, the routine organizes and lists the patients based on the group, then the total weight factor for the patient, and then last name of the patient.

Spatial and functional relationships between elements (for example, between modules) are described using various terms, including "connected," "engaged," "interfaced," and "coupled." Unless explicitly described as being "direct," when a relationship between first and second elements is described in the above disclosure, that relationship encompasses a direct relationship where no other intervening elements are present between the first and second elements, and also an indirect relationship where one or more intervening elements are present (either spatially or functionally) between the first and second elements. As used herein, the phrase at least one of A, B, and C should be construed to mean a logical (A OR B OR C), using a non-exclusive logical OR, and should not be construed to mean "at least one of A, at least one of B, and at least one of C."

In this application, including the definitions below, the term 'module' or the term 'controller' may be replaced with the term 'circuit.' The term 'module' may refer to, be part of, or include processor hardware (shared, dedicated, or group) that executes code and memory hardware (shared, dedicated, or group) that stores code executed by the processor hardware.

The module may include one or more interface circuits. In some examples, the interface circuits may include wired or wireless interfaces that are connected to a local area network (LAN), the Internet, a wide area network (WAN), or combinations thereof. The functionality of any given module of the present disclosure may be distributed among multiple modules that are connected via interface circuits. For example, multiple modules may allow load balancing. In a further example, a server (also known as remote, or cloud) module may accomplish some functionality on behalf of a client module.

The term code, as used above, may include software, firmware, and/or microcode, and may refer to programs, routines, functions, classes, data structures, and/or objects. Shared processor hardware encompasses a single microprocessor that executes some or all code from multiple modules. Group processor hardware encompasses a microprocessor that, in combination with additional microprocessors, executes some or all code from one or more modules. References to multiple microprocessors encompass multiple microprocessors on discrete dies, multiple microprocessors on a single die, multiple cores of a single microprocessor, multiple threads of a single microprocessor, or a combination of the above.

Shared memory hardware encompasses a single memory device that stores some or all code from multiple modules. Group memory hardware encompasses a memory device that, in combination with other memory devices, stores some or all code from one or more modules.

The term memory hardware is a subset of the term computer-readable medium. The term computer-readable medium, as used herein, does not encompass transitory electrical or electromagnetic signals propagating through a medium (such as on a carrier wave); the term computer-readable medium is therefore considered tangible and non-transitory. Non-limiting examples of a non-transitory computer-readable medium are nonvolatile memory devices (such as a flash memory device, an erasable programmable read-only memory device, or a mask read-only memory device), volatile memory devices (such as a static random access memory device or a dynamic random access memory device), magnetic storage media (such as an analog or digital magnetic tape or a hard disk drive), and optical storage media (such as a CD, a DVD, or a Blu-ray Disc).

The apparatuses and methods described in this application may be partially or fully implemented by a special purpose computer created by configuring a general purpose computer to execute one or more particular functions embodied in computer programs. The functional blocks and flowchart elements described above serve as software specifications, which can be translated into the computer programs by the routine work of a skilled technician or programmer.

The computer programs include processor-executable instructions that are stored on at least one non-transitory computer-readable medium. The computer programs may also include or rely on stored data. The computer programs may encompass a basic input/output system (BIOS) that interacts with hardware of the special purpose computer, device drivers that interact with particular devices of the special purpose computer, one or more operating systems, user applications, background services, background applications, etc.

The computer programs may include: (i) descriptive text to be parsed, such as HTML (hypertext markup language) or XML (extensible markup language), (ii) assembly code, (iii) object code generated from source code by a compiler, (iv) source code for execution by an interpreter, (v) source code for compilation and execution by a just-in-time compiler, etc. As examples only, source code may be written using syntax from languages including C, C++, C#, Objective-C, Haskell, Go, SQL, R, Lisp, Java^{®}, Fortran, Perl, Pascal, Curl, OCaml, Javascript^{®}, HTML5, Ada, ASP (active server pages), PHP, Scala, Eiffel, Smalltalk, Erlang, Ruby, Flash^{®}, Visual Basic^{®}, Lua, and Python^{®}.

## Claims

1. A computer implemented method for prioritizing patients associated with a healthcare provider, comprising:
- determining, by a diabetes management system, one or more glucose conditions for each patient in a plurality of patients associated with the healthcare provider, wherein each of the one or more glucose conditions is indicative of a glucose level of the patient and is assigned a condition weight, wherein the one or more glucose conditions are selected from a group consisting of a hypoglycemic condition, a hyperglycemic condition and a variability condition;
- determining, by the diabetes management system, a state for each glucose condition associated with a given patient using an alert threshold,
wherein:
- the state for a given glucose condition is selected from a group consisting of a HIGH state, a LOW state, and an unavailable state,
- the LOW state indicates that the glucose condition is within an acceptable range,
- the HIGH state indicates that the glucose condition is outside the acceptable range, and
- the unavailable state indicates insufficient data is available in the enquiry period to determine the state of the glucose condition,
- each state is assigned a state weight, the alert threshold is indicative of an acceptable range for the glucose condition, and the determination of a state is made for each patient in the plurality of patients;
- determining, by the diabetes management system, a total alert value for each patient in the plurality of patients by summing together the product of the condition weight and the state weight for each of glucose condition associated with a given patient; and
- displaying, by the diabetes management system, a listing of the patients on a display of a computing device, where the patients are arranged in accordance with the total alert value, wherein the patients in the listing of the patients are arranged in descending order in accordance with the total alert value.

2. The computer implemented method of claim 1, wherein each entry in the listing of patients includes a name for the given patient and indicia for the state of the one or more glucose conditions associated with the given patient.

3. The computer implemented method of claim 1, wherein value of the condition weight assigned to the hypoglycemic condition is greater than value of the condition weight assigned to the hyperglycemic condition and value of the condition weight assigned to the hyperglycemic condition is greater than the value of the condition weight assigned to the variability condition.

4. The computer implemented method of claim 1, wherein value of the state weight assigned to the HIGH state is greater than value of the state weight assigned to the LOW state, and value of the state weight assigned to the LOW state is greater than the value of the state weight assigned to the unavailable state.

5. The computer implemented method of claim 1, wherein determining one or more glucose conditions further comprises:
- receiving, by the diabetes management system, an enquiry period within which an alert status for the patient is evaluated;
- retrieving, by the diabetes management system, glucose measurements for the patient that fall within the enquiry period from a database; and
- for each glucose measurements that falls within the enquiry period, determining, by the diabetes management system, the glucose condition of the patient by comparing a given glucose measurement to a glucose threshold, wherein the glucose condition is indicative of a glucose level of the patient and the glucose threshold represents a satisfactory range for the given glucose measurement.

6. The computer implemented method of claim 5, wherein determining state for the glucose condition further comprises
- determining a number of occurrences of hypoglycemic condition in the enquiry period, where an occurrence of a hypoglycemic condition equates to a given glucose measurement being less than a glucose threshold for a hypoglycemic condition;
- determining a total number of glucose measurements during the enquiry period;
- dividing the number of occurrences of the hypoglycemic condition by the total number of glucose measurements to yield a quotient; and
- comparing the quotient to an alert threshold for a hypoglycemic condition.

7. The computer implemented method of claim 5, wherein determining state for the glucose condition further comprises:
- determining a number of occurrences of a hyperglycemic condition in the enquiry period, where the occurrence of the hyperglycemic condition equates to a given glucose measurement being more than a glucose threshold for the hyperglycemic condition;
- determining a total number of glucose measurements during the enquiry period;
- dividing the number of occurrences of the hyperglycemic condition by the total number of glucose measurements to yield a quotient; and
- comparing the quotient to an alert threshold for the hyperglycemic condition.

8. The computer implemented method of claim 5, wherein determining state for the glucose condition further comprises:
- determining a standard deviation for the glucose measurements that fall within the enquiry period;
- determining a mean for the glucose measurements that fall within the enquiry period;
- dividing the standard deviation by the mean to yield a quotient; and
- comparing the quotient to an alert threshold for a variability condition.

## Patentansprüche

1. Computerimplementiertes Verfahren zum Priorisieren von Patienten in Verbindung mit einem Gesundheitsdienstleister, umfassend:
- Bestimmen eines Glukosezustands oder mehrerer Glukosezustände für jeden Patienten von einer Vielzahl von Patienten in Verbindung mit dem Gesundheitsdienstleister mittels eines Diabetesmanagementsystems, wobei jeder der ein oder mehreren Glukosezustände einen Glukosespiegel des Patienten angibt und einer Zustandswichtung zugeordnet ist, wobei der eine Glukosezustand oder die mehreren Glukosezustände aus einer Gruppe ausgewählt ist/sind, die aus einem hypoglykämischem Zustand, einem hyperglykämischen Zustand und einem Variabilitätszustand besteht;
- Bestimmen eines Status für jeden Glukosezustand in Verbindung mit einem gegebenen Patienten unter Verwendung einer Alarmschwelle mittels des Diabetesmanagementsystems,
wobei:
- der Status für einen gegebenen Glukosezustand aus einer Gruppe ausgewählt ist, die aus einem HOCH-Status, einem NIEDRIG-Status und einem nicht-verfügbar-Status besteht,
- der NIEDRIG-Status angibt, dass der Glukosezustand innerhalb eines akzeptablen Bereiches liegt,
- der HOCH-Status angibt, dass der Glukosezustand außerhalb des akzeptablen Bereiches liegt und
- der nicht-verfügbar-Status angibt, dass nicht ausreichend Daten in der Abfragedauer zum Bestimmen des Status des Glukosezustands zur Verfügung stehen,
- jeder Status einer Statuswichtung zugeordnet ist, wobei die Alarmschwelle einen akzeptablen Bereich für den Glukosezustand angibt und die Bestimmung eines Status für jeden Patienten von der Vielzahl von Patienten erfolgt;
- Bestimmen eines Gesamtalarmwertes für jeden Patienten von der Vielzahl von Patienten mittels des Diabetesmanagementsystems durch Aufsummieren des Produktes der Zustandswichtung und der Statuswichtung für jeden Glukosezustand in Verbindung mit einem gegebenen Patienten; und
- Anzeigen einer Auflistung der Patienten auf einer Anzeige einer Rechenvorrichtung mittels des Diabetesmanagementsystems, wo die Patienten gemäß dem Gesamtalarmwert angeordnet sind, wobei die Patienten in der Auflistung der Patienten absteigend gemäß dem Gesamtalarmwert angeordnet sind.

2. Computerimplementiertes Verfahren nach Anspruch 1, wobei jede Eingabe in die Auflistung von Patienten einen Namen für den gegebenen Patienten und Indikatoren für den Status des einen Glukosezustands oder der mehreren Glukosezustände in Verbindung mit dem gegebenen Patienten einschließt.

3. Computerimplementiertes Verfahren nach Anspruch 1, wobei ein Wert der Zustandswichtung, die dem hypoglykämischen Zustand zugeordnet ist, größer ist als ein Wert der Zustandswichtung, die dem hyperglykämischen Zustand zugeordnet ist, und ein Wert der Zustandswichtung, die dem hyperglykämischen Zustand zugeordnet ist, größer ist als der Wert der Zustandswichtung, die dem Variabilitätszustand zugeordnet ist.

4. Computerimplementiertes Verfahren nach Anspruch 1, wobei ein Wert der Statuswichtung, die dem HOCH-Status zugeordnet ist, größer ist als ein Wert der Statuswichtung, die dem NIEDRIG-Status zugeordnet ist, und ein Wert der Statuswichtung, die dem NIEDRIG-Status zugeordnet ist, größer ist als der Wert der Statuswichtung, die dem nicht-verfügbar-Status zugeordnet ist.

5. Computerimplementiertes Verfahren nach Anspruch 1, wobei das Bestimmen eines Glukosezustands oder mehrerer Glukosezustände ferner Folgendes umfasst:
- Empfangen einer Abfragedauer, innerhalb der ein Alarmstatus für den Patienten ausgewertet wird, mittels des Diabetesmanagementsystems;
- Abrufen von Glukosemessungen für den Patienten, die in die Abfragedauer fallen, aus einer Datenbank mittels des Diabetesmanagementsystems; und
- für jede Glukosemessung, die in die Abfragedauer fällt, Bestimmen des Glukosezustands des Patienten mittels des Diabetesmanagementsystems, indem eine gegebene Glukosemessung mit einer Glukoseschwelle verglichen wird, wobei der Glukosezustand einen Glukosespiegel des Patienten angibt und die Glukoseschwelle einen zufriedenstellenden Bereich für die gegebene Glukosemessung darstellt.

6. Computerimplementiertes Verfahren nach Anspruch 5, wobei das Bestimmen des Status für den Glukosezustand ferner Folgendes umfasst
- Bestimmen einer Anzahl von Vorkommnissen eines hypoglykämischen Zustands in der Abfragedauer, wobei ein Vorkommnis eines hypoglykämischen Zustands damit gleichzusetzen ist, dass eine gegebene Glukosemessung weniger beträgt als eine Glukoseschwelle für einen hypoglykämischen Zustand;
- Bestimmen einer Gesamtanzahl von Glukosemessungen während der Abfragedauer;
- Teilen der Anzahl von Vorkommnissen des hypoglykämischen Zustands durch die Gesamtanzahl von Glukosemessungen, was einen Quotienten ergibt; und
- Vergleichen des Quotienten mit einer Alarmschwelle für einen hypoglykämischen Zustand.

7. Computerimplementiertes Verfahren nach Anspruch 5, wobei das Bestimmen des Status für den Glukosezustand ferner Folgendes umfasst:
- Bestimmen einer Anzahl von Vorkommnissen eines hyperglykämischen Zustands in der Abfragedauer, wobei das Vorkommnis des hyperglykämischen Zustands damit gleichzusetzen ist, dass eine gegebene Glukosemessung mehr beträgt als eine Glukoseschwelle für den hyperglykämischen Zustand;
- Bestimmen einer Gesamtanzahl von Glukosemessungen während der Abfragedauer;
- Teilen der Anzahl von Vorkommnissen des hyperglykämischen Zustands durch die Gesamtanzahl von Glukosemessungen, was einen Quotienten ergibt; und
- Vergleichen des Quotienten mit einer Alarmschwelle für den hyperglykämischen Zustand.

8. Computerimplementiertes Verfahren nach Anspruch 5, wobei das Bestimmen des Status für den Glukosezustand ferner Folgendes umfasst:
- Bestimmen einer Standardabweichung für die Glukosemessungen, die in die Abfragedauer fallen;
- Bestimmen eines Mittelwertes für die Glukosemessungen, die in die Abfragedauer fallen;
- Teilen der Standardabweichung durch den Mittelwert, was einen Quotienten ergibt; und
- Vergleichen des Quotienten mit einer Alarmschwelle für einen Variabilitätszustand.

## Revendications

1. Procédé mis en œuvre par ordinateur pour la priorisation de patients associés à un prestataire de soins de santé, comprenant :
- la détermination, par un système de gestion du diabète, d'une ou plusieurs conditions de glycémie pour chaque patient parmi une pluralité de patients associés au prestataire de soins de santé, dans lequel chacune des une ou plusieurs conditions de glycémie indique un taux de glycémie du patient et se voit attribuer un poids de condition, dans lequel la ou les conditions de glycémie sont choisies dans un groupe constitué par une condition hypoglycémique, une condition hyperglycémique et une condition de variabilité ;
- la détermination, par le système de gestion du diabète, d'un état pour chaque
condition de glycémie associée à un patient donné en utilisant un seuil d'alerte, dans lequel :
- l'état pour une condition de glycémie donnée est choisi dans un groupe constitué par un état ÉLEVÉ, un état FAIBLE et un état indisponible,
- l'état FAIBLE indique que la condition de glycémie est dans une plage acceptable,
- l'état ÉLEVÉ indique que la condition de glycémie est en dehors de la plage acceptable, et
- l'état indisponible indique que les données disponibles au cours de la période d'investigation sont insuffisantes pour déterminer l'état de la condition de glycémie,
- chaque état se voit attribuer un poids d'état, le seuil d'alerte indique une plage acceptable pour la condition de glycémie, et la détermination d'un état est réalisée pour chaque patient parmi la pluralité de patients ;
- la détermination, par le système de gestion du diabète, d'une valeur d'alerte totale pour chaque patient parmi la pluralité de patients en additionnant le produit du poids de condition et le poids d'état pour chacune des conditions de glycémie associées à un patient donné ; et
- l'affichage, par le système de gestion du diabète, d'une liste des patients sur un écran d'un dispositif informatique, où les patients sont classés en fonction de la valeur d'alerte totale, dans lequel les patients de la liste des patients sont classés dans un ordre décroissant en fonction de la valeur d'alerte totale.

2. Procédé mis en œuvre par ordinateur selon la revendication 1, dans lequel chaque entrée de la liste de patients comprend un nom pour le patient donné et des indices pour l'état de la ou des conditions de glycémie associées au patient donné.

3. Procédé mis en œuvre par ordinateur selon la revendication 1, dans lequel la valeur du poids de condition attribué à la condition hypoglycémique est supérieure à la valeur du poids de condition attribué à la condition hyperglycémique et la valeur du poids de condition attribué à la condition hyperglycémique est supérieure à la valeur du poids de condition attribué à la condition de variabilité.

4. Procédé mis en œuvre par ordinateur selon la revendication 1, dans lequel la valeur du poids d'état attribué à l'état ÉLEVÉ est supérieure à la valeur du poids d'état attribué à l'état FAIBLE, et la valeur du poids d'état attribué à l'état FAIBLE est supérieure à la valeur du poids d'état attribué à l'état indisponible.

5. Procédé mis en œuvre par ordinateur selon la revendication 1, dans lequel la détermination d'une ou plusieurs conditions de glycémie comprend en outre :
- la réception, par le système de gestion du diabète, d'une période d'investigation au cours de laquelle un état d'alerte pour le patient est évalué ;
- la récupération, par le système de gestion du diabète, de mesures de glycémie pour le patient qui se situent dans la période d'investigation d'une base de données ; et
- pour chacune des mesures de glycémie qui se situe dans la période d'investigation, la détermination, par le système de gestion du diabète, de la condition de glycémie du patient en comparant une mesure de glycémie donnée à un seuil de glycémie, dans lequel la condition de glycémie indique un taux de glycémie du patient et le seuil de glycémie représente une plage satisfaisante pour la mesure de glycémie donnée.

6. Procédé mis en œuvre par ordinateur selon la revendication 5, dans lequel la détermination de l'état pour la condition de glycémie comprend en outre
- la détermination d'un nombre d'occurrences de condition hypoglycémique dans la période d'investigation, où une occurrence d'une condition hypoglycémique équivaut à une mesure de glycémie donnée inférieure à un seuil de glycémie pour une condition hypoglycémique ;
- la détermination d'un nombre total de mesures de glycémie pendant la période d'investigation ;
- la division du nombre d'occurrences de la condition hypoglycémique par le nombre total de mesures de glycémie pour donner un quotient ; et
- la comparaison du quotient avec un seuil d'alerte pour une condition hypoglycémique.

7. Procédé mis en œuvre par ordinateur selon la revendication 5, dans lequel la détermination de l'état pour la condition de glycémie comprend en outre :
- la détermination d'un nombre d'occurrences d'une condition hyperglycémique dans la période d'investigation, où l'occurrence de la condition hyperglycémique équivaut à une mesure de glycémie donnée supérieure à un seuil de glycémie pour la condition hyperglycémique ;
- la détermination d'un nombre total de mesures de glycémie pendant la période d'investigation ;
- la division du nombre d'occurrences de la condition hyperglycémique par le nombre total de mesures de glycémie pour donner un quotient ; et
- la comparaison du quotient avec un seuil d'alerte pour la condition hyperglycémique.

8. Procédé mis en œuvre par ordinateur selon la revendication 5, dans lequel la détermination de l'état pour la condition de glycémie comprend en outre :
- la détermination d'un écart-type pour les mesures de glycémie qui se situent dans la période d'investigation ;
- la détermination d'une moyenne pour les mesures de glycémie qui se situent dans la période d'investigation ;
- la division de l'écart-type par la moyenne pour donner un quotient ; et
- la comparaison du quotient avec un seuil d'alerte pour une condition de variabilité.
